# EUROPÄISCHE PATENTANMELDUNG

(11) **EP 1 493 821 A1**
(43) Veröffentlichungstag der Anmeldung: **05.01.2005**
(21) Anmeldenummer: 04014648.2
(22) Anmeldetag: 23.06.2004
(51) Int. Cl.: C12Q 1/18

(54) **Verfahren zum Identifizieren von fungizid wirksamen Verbindungen basierend auf Guanylat Kinasen**

(30) Priorität: 04.07.2003 DE 10330233
(71) Anmelder: Bayer CropScience AG, 40789 Monheim (DE)
(72) Erfinder: Vaupel, Martin, Dr., 42799 Leichlingen (DE); Koopmann, Edda, Dr., 42799 Leichlingen (DE); Vollenbroich, Verena, Dr., 45478 Mühlheim (DE)

(57) **Zusammenfassung**

Die Erfindung bezieht sich auf ein Verfahren zum Identifizieren von Fungiziden, die Verwendung von pilzlicher Guanylat Kinase zum Identifizieren von Fungiziden, und die Verwendung von Inhibitoren der Guanylat Kinase als Fungizide.

### SEQUENCE LISTING

<110> Bayer CropScience AG
<120> Verfahren zum Identifizieren von fungizid wirksamen Verbindungen basierend auf Guanylat Kinasen
<130> BCS 03-3046
<160> 2
<170> PatentIn version 3.1
<210> 1
   <211> 609
   <212> DNA
   <213> Ustilago maydis
<220><
   <221> CDS
   <222> (1)..(609)
   <223>
<400> 1
   atg ccg tcg tcc aac tgg gcg tcg acg cct ccg att cca agc gac aaa      48
   Met Pro Ser Ser Asn Trp Ala Ser Thr Pro Pro Ile Pro Ser Asp Lys
   1               5                   10                  15
   cgt ccg atc gtg ctg tcg ggt cct tcg ggt gtg ggc aag agt aca ctt      96
   Arg Pro Ile Val Leu Ser Gly Pro Ser Gly Val Gly Lys Ser Thr Leu
               20                  25                  30
   ctg aaa aag ttg ttt caa gag ttc ccc aat gac ttt ggt ttt tcc gta     144
   Leu Lys Lys Leu Phe Gln Glu Phe Pro Asn Asp Phe Gly Phe Ser Val
           35                  40                  45
   tct gat aca aca aga gac cct cga cca ggc gag gtc cgc ggc cag tcg     192
   Ser Asp Thr Thr Arg Asp Pro Arg Pro Gly Glu Val Arg Gly Gln Ser
       50                  55                  60
   tac cac tac gtc tca cag caa gaa ttc caa gac ctt gtt caa cag ggc     240
   Tyr His Tyr Val Ser Gln Gln Glu Phe Gln Asp Leu Val Gln Gln Gly
   65                  70                  75                  80
   gcg ttt ctc gaa cac gcc aag ttt ggc ggc aac cga tac ggc act acc     288
   Ala Phe Leu Glu His Ala Lys Phe Gly Gly Asn Arg Tyr Gly Thr Thr
                   85                  90                  95
   gcc aag gcg gtg gct gac gta tcc acc gaa ggc cgt cgc gct atc ctc     336
   Ala Lys Ala Val Ala Asp Val Ser Thr Glu Gly Arg Arg Ala Ile Leu
               100                 105                 110
   gac atc gac gct caa ggc gtc aag ctc atc aag gcc aac cac gct tcg     384
   Asp Ile Asp Ala Gln Gly Val Lys Leu Ile Lys Ala Asn His Ala Ser
           115                 120                 125
   ttg aac ccg atc tac atc ttt atc tct cca cct tcg ttt tcg act ctc     432
   Leu Asn Pro Ile Tyr Ile Phe Ile Ser Pro Pro Ser Phe Ser Thr Leu
       130                 135                 140
   aag cag agg ttg ata ggt cga ggc acc gag aca cca gag tca gtc aac     480
   Lys Gln Arg Leu Ile Gly Arg Gly Thr Glu Thr Pro Glu Ser Val Asn
   145                 150                 155                 160
   aag agg ctc tct atg gct gcc acc gag atg gct tat gcc agg gaa aag     528
   Lys Arg Leu Ser Met Ala Ala Thr Glu Met Ala Tyr Ala Arg Glu Lys
                   165                 170                 175
   gga gcg cac gat tgg gtc att gtc aat gac gat ttg gaa aag gct tat     576
   Gly Ala His Asp Trp Val Ile Val Asn Asp Asp Leu Glu Lys Ala Tyr
               180                 185                 190
   ggc ttg ttg aag aag gcg atc aac gaa act ttg                         609
   Gly Leu Leu Lys Lys Ala Ile Asn Glu Thr Leu
           195                 200
<210> 2
   <211> 203
   <212> PRT
   <213> Ustilago maydis
<400> 2
   Met Pro Ser Ser Asn Trp Ala Ser Thr Pro Pro Ile Pro Ser Asp Lys
   1               5                   10                  15
   Arg Pro Ile Val Leu Ser Gly Pro Ser Gly Val Gly Lys Ser Thr Leu
               20                  25                  30
   Leu Lys Lys Leu Phe Gln Glu Phe Pro Asn Asp Phe Gly Phe Ser Val
           35                  40                  45
   Ser Asp Thr Thr Arg Asp Pro Arg Pro Gly Glu Val Arg Gly Gln Ser
       50                  55                  60
   Tyr His Tyr Val Ser Gln Gln Glu Phe Gln Asp Leu Val Gln Gln Gly
   65                  70                  75                  80
   Ala Phe Leu Glu His Ala Lys Phe Gly Gly Asn Arg Tyr Gly Thr Thr
                   85                  90                  95
   Ala Lys Ala Val Ala Asp Val Ser Thr Glu Gly Arg Arg Ala Ile Leu
               100                 105                 110
   Asp Ile Asp Ala Gln Gly Val Lys Leu Ile Lys Ala Asn His Ala Ser
           115                 120                 125
   Leu Asn Pro Ile Tyr Ile Phe Ile Ser Pro Pro Ser Phe Ser Thr Leu
       130                 135                 140
   Lys Gln Arg Leu Ile Gly Arg Gly Thr Glu Thr Pro Glu Ser Val Asn
   145                 150                 155                 160
   Lys Arg Leu Ser Met Ala Ala Thr Glu Met Ala Tyr Ala Arg Glu Lys
                   165                 170                 175
   Gly Ala His Asp Trp Val Ile Val Asn Asp Asp Leu Glu Lys Ala Tyr
               180                 185                 190
   Gly Leu Leu Lys Lys Ala Ile Asn Glu Thr Leu
           195                 200

## Beschreibung

Die Erfindung bezieht sich auf ein Verfahren zum Identifizieren von Fungiziden, die Verwendung von pilzlicher Guanylat Kinase zum Identifizieren von Fungiziden, und die Verwendung von Inhibitoren der Guanylat Kinase als Fungizide.

Unerwünschtes Pilzwachstum, das in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben.

Aufgabe der vorliegenden Erfindung war es deshalb, einen geeigneten neuen Angriffspunkt für potentielle fungizide Wirkstoffe zu identifizieren und zugänglich zu machen, und ein Verfahren zur Verfügung zu stellen, das die Identifizierung von Modulatoren dieses Angriffspunkts ermöglicht, die dann als Fungizide verwendet werden können.

Die Guanylat Kinase (EC 2.7.4.8), auch bekannt als Deoxyguanylat Kinase, 5'-GMP Kinase, GMP Kinase, Guanosinmonophosphat Kinase, ATP:GMP Phosphotransferase, katalysiert die Reaktion von ATP und GMP zu ADP und GDP. Die Reaktion der Guanylat Kinase ist ein essentieller Schritt im Recycling von GMP bzw. in der Herstellung von GTP (Li et al., 1996).

Gene für die Guanylat Kinase wurden aus verschiedenen Organismen kloniert, darunter auch aus verschiedenen Pilzen, z.B. aus *Saccharomyces cerevisiae* (Swissprot Accession No.: P15454), *Neurospora crassa* (Genbank Accession No. AABX01000205), *Schizosaccharomyces pombe* (Swissprot Accession No.: Q9P6I5), der Maus (Swissprot Accession No.: Q64520) oder *Homo sapiens* (Swissprot: Accession No.: Q16774). Die Sequenzähnlichkeiten sind innerhalb der eukaryontischen Klassen signifikant.

Die Guanylat Kinase wurde bereits z.B. aus Hefe isoliert, exprimiert, gereinigt, charakterisiert und kristallisiert (Moriguchi et al., 1981; Berger et al., 1989; Stehle et al., 1990; Konrad, 1992; Blaszczyk et al., 2001).

Aufgabe der vorliegenden Erfindung war es, neue Angriffspunkte von Fungiziden in Pilzen, insbesondere in phytopathogenen Pilzen, zu identifizieren und ein Verfahren zugänglich zu machen, in denen Inhibitoren eines solchen Angriffspunktes bzw. Polypeptids identifiziert und auf ihre fungiziden Eigenschaften hin geprüft werden können. Die Aufgabe wurde gelöst, indem aus einem phytopathogenen Pilz die für Guanylat Kinase kodierende Nukleinsäure isoliert, das davon kodierte Polypeptid gewonnen und ein Verfahren zur Verfügung gestellt wurde, mit dem Inhibitoren dieses Enzyms bestimmt werden können. Die mit diesem Verfahren identifizierten Inhibitoren können *in vivo* gegen Pilze eingesetzt werden.

### Beschreibung der Abbildungen

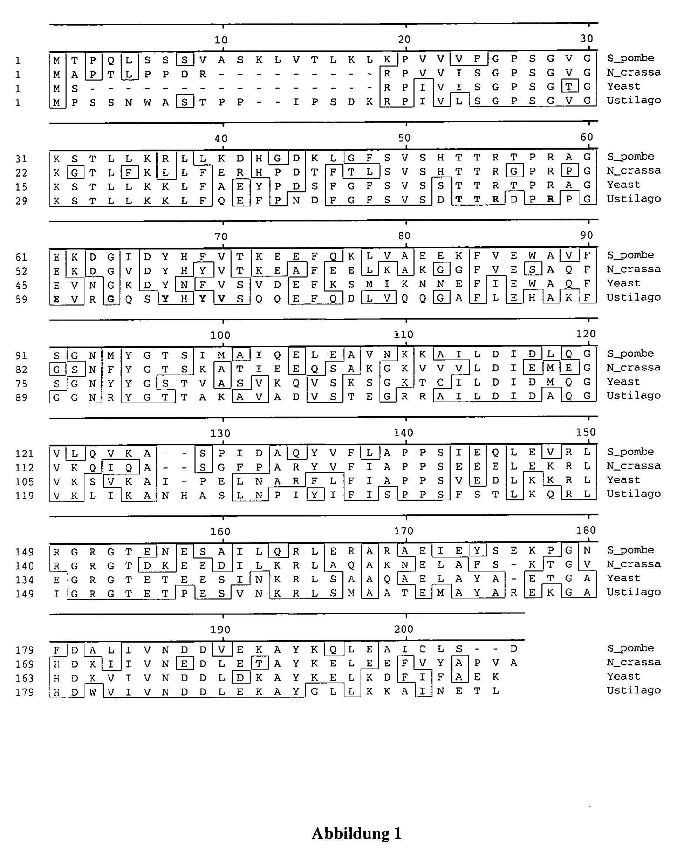

- **Abbildung 1:**: Homologie zwischen Guanylat Kinasen aus verschiedenen Pilzen. Rahmen geben Bereiche mit einer genau übereinstimmenden Sequenz wieder (Konsensussequenz). In der Sequenz aus *Ustilago maydis* wurden weiterhin die dem Prosite Motiv für Guanylat Kinasen entsprechenden Aminosäuren gekennzeichnet. S_pombe: *Saccharomyces pombe.* N_crassa: *Neurospora crassa*. Yeast: *Saccharomyces cerevisiae.* Ustilago: *Ustilago maydis*.

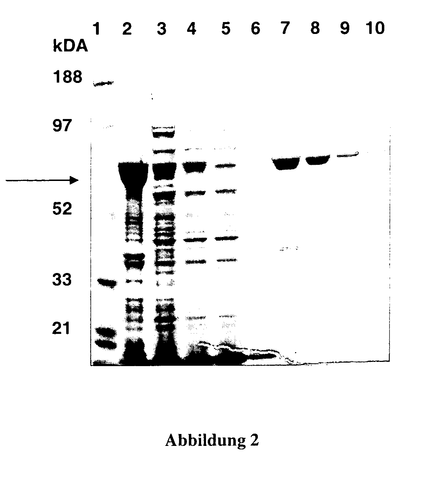

- **Abbildung 2:**: Heterologe Expression der Guanylat Kinase in *E. coli* BL21(DE3) pLysS. Das überexprimierte MBP-Fusionsprotein hat eine Größe von ca. 64 kDa. In Spur 1 wurde ein Größenstandard aufgetragen. Spur 2: Membranfraktion 4 Stunden nach der Induktion mit IPTG, Zellaufschluss und Trennung von Membran- und Cytoplasmafraktion mittels Zentrifugation; Spur 3: Cytoplasmafraktion der überexprimierten Guanylat Kinase 4 Stunden nach der Induktion mit IPTG, Zellaufschluss und Trennung von Membran- und Cytoplasmafraktion mittels Zentrifugation; Spur 4: Cytoplasmafraktion der überexprimierten Guanylat Kinase 4 Stunden nach der Induktion mit IPTG, Zellaufschluss und Trennung von Membran- und Cytoplasmafraktion mittels Zentrifugation, jedoch 1:1 mit Puffer verdünnt; Spur 5: Durchfluss bei der Auftragung der Cytoplasmafraktion auf das Amylose Resin; Spur 6: Waschfraktionen nach dem Auftragen der Cytoplasmafraktion auf das Amylose Resin; Spur 7-10: Elutionsfraktionen mit gereinigter Guanylat Kinase.

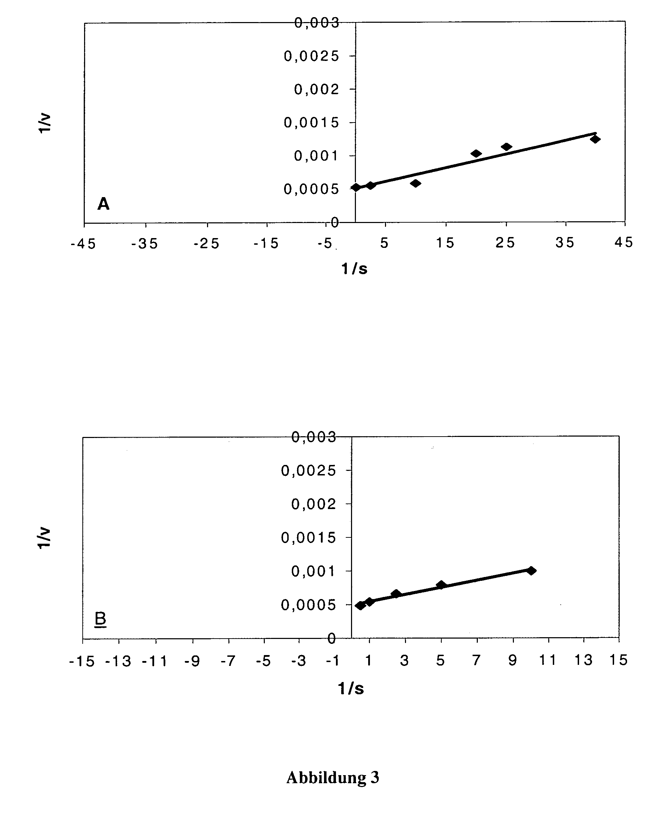

- **Abbildung 3:**: K_{M}-Wert Bestimmung für GMP (A) und ATP (B). Darstellung der gemessenen Werte nach Lineweaver und Burk: 1/Vₒ = 1/Vₘₐₓ + 1/S x (K_{M}/Vₘₐₓ), worin Vₒ die anfängliche Reaktionsgeschwindigkeit, Vₘₐₓ die maximal erreichbare Umsatzgeschwindigkeit und S die Substratkonzentarion ist. Vₘₐₓ und K_{M} lassen sich dann als Abszissen- und Ordinatenabschnitt 1/Vₘₐₓ bzw. 1/K_{M} ablesen. Der K_{M}-Wert für GMP beträgt 0,04 mM, für ATP 0,1 mM.
- **SEQ ID NO:1**: Nukleinsäuresequenz kodierend für die Guanylat Kinase aus *Ustilago maydis*.
- **SEQ ID NO: 2**: Aminosäuresequenz der Guanylat Kinase aus *Ustilago maydis.*

### Definitionen

Unter dem Begriff "Homologie" bzw. "Identität" soll die Anzahl der übereinstimmenden Aminosäuren (Identität) mit anderen Proteinen, ausgedrückt in Prozent verstanden werden. Bevorzugt wird die Identität durch Vergleiche einer gegebenen Sequenz zu anderen Proteinen mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Die Identität kann standardmäßig mittels bekannten und der Öffentlichkeit zur Verfügung stehenden Computerprogrammen wie z.B. ClustalW (Thompson et al., *Nucleic Acids Research 22* (1994), 4673-4680) ermittelt werden. ClustalW wird z.B. öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Intemetseiten des EBI (European Bioinformatics Institute, Wellcome Trust Genome Campus, Hinxton, Cambridge CB10 1SD, UK), heruntergeladen werden. Wenn das ClustalW Computerprogramm der Version 1.8 benutzt wird, um die Identität zwischen z.B. einem gegebenen Referenzprotein und anderen Proteinen zu bestimmen, sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP. Eine Möglichkeit zum Auffinden von ähnlichen Sequenzen ist die Durchführung von Sequenzdatenbankrecherchen. Hierbei wird eine oder werden mehrere Sequenzen als sogenannte Abfrage ("query") vorgegeben. Diese Abfragesequenz wird dann mittels statistischen Computerprogrammen mit Sequenzen, die in den ausgewählten Datenbanken enthalten sind, verglichen. Solche Datenbankabfragen ("blast searches") sind dem Fachmann bekannt und können bei verschiedenen Anbietern durchgeführt werden. Wird eine solche Datenbankabfrage z.B. beim NCBI (National Center for Biotechnology Information, http://www.ncbi.nlm.nih.gov/) durchgeführt, so sollen die Standardeinstellungen, die für die jeweilige Vergleichsanfrage vorgegeben sind, benutzt werden. Für Proteinsequenzvergleiche ("blastp") sind dieses folgende Einstellungen: Limit entrez = nicht aktiviert; Filter = low complexity aktiviert; Expect value = 10; word size = 3; Matrix = BLOSUM62; Gap costs: Existence = 11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt. Unter einem erfindungsgemäßen Protein sollen daher im Zusammenhang mit der vorliegenden Erfindung solche Proteine verstanden werden, die bei der Verwendung mindestens einer der vorstehend beschriebenen Methoden zur Identitätsbestimmung eine Identität von mindestens 70 % aufweisen, bevorzugt von mindestens 75 %, besonders bevorzugt von mindestens 80 %, weiter bevorzugt von mindestens 85 %, und insbesondere von mindestens 90 %.

Der Ausdruck "vollständige Guanylat Kinase" wie er hierin verwendet wird, beschreibt eine Guanylat Kinase, die von einer vollständigen kodierenden Region einer Transkriptionseinheit kodiert wird, umfassend ein ATG-Startcodon und umfassend alle informationstragenden Exonbereiche des im Herkunftsorganismus vorliegenden, für eine Guanylat Kinase kodierenden Gens, sowie die für eine korrekte Termination der Transkription nötigen Signale.

Der Ausdruck "biologische Aktivität einer Guanylat Kinase" wie er hierin verwendet wird, bezieht sich auf die Fähigkeit eines Polypeptids, die vorstehend beschriebene. Reaktion, d.h. die Umsetzung von ATP und GMP zu ADP und GDP zu katalysieren (ATP + GMP ⇔ ADP + GDP). Charakteristisch für Guanylat Kinasen ist ein GMP-Bindungsmotiv.

Der Ausdruck "aktives Fragment" wie er hierin verwendet wird, beschreibt nicht mehr vollständige Nukleinsäuren kodierend für Guanylat Kinase, die aber noch für Polypeptide mit der biologischen Aktivität einer Guanylat Kinase kodieren, und die eine für die Guanylat Kinase charakteristische Reaktion wie vorne beschrieben katalysieren können. Solche Fragmente sind kürzer als die oben beschriebenen vollständigen, für die Guanylat Kinase kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der Guanylat Kinase nicht entscheidend beeinträchtigen. Eine geringere oder gegebenenfalls auch eine erhöhte Aktivität, die aber noch die Charakterisierung bzw. Verwendung des resultierenden Guanylat Kinase Fragments gestattet, wird dabei als ausreichend im Sinne des hier verwendeten Ausdrucks verstanden. Der Ausdruck "aktives Fragment" kann sich ebenso auf die Aminosäuresequenz der Guanylat Kinase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die Fragmente können dabei verschiedene Länge besitzen.

Der Begriff "Guanylat Kinase-Hemmtest" oder "Hemmtest", wie er hierin verwendet wird, bezieht sich auf ein Verfahren bzw. einen Test, der es gestattet, die Inhibition der enzymatischen Aktivität eines Polypeptids mit der Aktivität einer Guanylat Kinase durch eine oder mehrere chemische Verbindungen (Kandidatenverbindung(en)) zu erkennen, wodurch die chemische Verbindung als Inhibitor der Guanylat Kinase identifiziert werden kann.

Der Ausdruck "Gen", wie er hierin verwendet wird, ist die Bezeichnung für einen Abschnitt aus dem Genom einer Zelle, die für die Synthese einer Polypeptid-Kette verantwortlich ist.

Der Ausdruck "Fungizid" bzw. "fungizid" wie er hierin verwendet wird, bezieht sich auf chemische Verbindungen, die zur Bekämpfung von Pilzen, insbesondere von pflanzenpathogenen Pilzen geeignet sind. Solche pflanzenpathogene Pilze werden nachfolgenden genannt, wobei die Aufzählung nicht abschließend ist:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum*, Phytophthora-Arten, wie beispielsweise *Phytophthora infestans*, Pseudoperonospora-Arten, wie beispielsweise *Pseudoperonospora humuli* oder *Pseudoperonospora cubensis,* Plasmopara-Arten, wie beispielsweise *Plasmopara viticola*, Bremia-Arten, wie beispielsweise *Bremia lactucae*, Peronospora-Arten, wie beispielsweise *Peronospora pisi* oder *P. brassicae*, Erysiphe-Arten, wie beispielsweise *Erysiphe graminis,* Sphaerotheca-Arten, wie beispielsweise *Sphaerotheca fuliginea*, Podosphaera-Arten, wie beispielsweise *Podosphaera leucotricha,* Venturia-Arten, wie beispielsweise *Venturia inaequalis*, Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus*, Puccinia-Arten, wie beispielsweise *Puccinia recondita*, Sclerotinia-Arten, wie beispielsweise *Sclerotinia sclerotiorum,* Tilletia-Arten, wie beispielsweise *Tilletia caries;* Ustilago-Arten, wie beispielsweise *Ustilago nuda* oder *Ustilago avenae*, Pellicularia-Arten, wie beispielsweise *Pellicularia sasakii*, Pyricularia-Arten, wie beispielsweise *Pyricularia oryzae*, Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum*, Cercospora-Arten, wie beispielsweise *Cercospora canescens,* Alternaria-Arten, wie beispielsweise *Alteniaria brassicae* oder Pseudocercosporella-Arten, wie beispielsweise *Pseudocercosporella herpotrichoides*.

Von besonderem Interesse sind z.B. auch *Magnaporthe grisea*, *Cochliobulus heterostrophus, Nectria hematococcus* and Phytophthora species.

Fungizide Wirkstoffe, die mit Hilfe der erfindungsgemäßen Guanylat Kinase gefunden werden, können aber auch mit Guanylat Kinase aus humanpathogenen Pilzspezies interagieren, wobei die Interaktion mit den unterschiedlichen in diesen Pilzen vorkommenden Guanylat Kinase nicht immer gleich stark sein muss.

Gegenstand der vorliegenden Erfindungen ist deshalb auch die Verwendung von Inhibitoren der Guanylat Kinase zum Herstellen von Mitteln zur Behandlung von durch humanpathogene Pilze hervorgerufenen Erkrankungen.

Dabei sind die folgenden humanpathogenen Pilze von besonderem Interesse, die die nachfolgend genannten Krankheitsbilder hervorrufen können:

Dermatophyten, wie z.B. Trichophyton spec., Microsporum spec., *Epidermophyton floccosum* oder *Keratomyces ajelloi*, die z.B. Fußmykosen (Tinea pedis) hervorrufen, Hefen, wie z.B. *Candida albicans*, Soor-Ösophagitis und Dermatitis hervorruft, *Candida glabrata, Candida krusei* oder *Cryptococcus neoformans*, die z.B. pulmonale Cryptococcose und auch Torulose hervorrufen können, Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis*, der z.B. subkutane Myzetome hervorruft, *Histoplasma capsulatum*, der z.B. retikuloendotheliale Zytomykose und M. Darling hervorruft, *Coccidioides immitis,* der z. B. pulmonale Coccidioidomykose und Sepsis hervorruft, *Paracoccidioides brasiliensis*, der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii*, der z.B. Sporotrichose (granulomatöse Hautmykose) hervorruft.

Fungizide Wirkstoffe, die mit Hilfe einer aus einem bestimmten Pilz, hier *aus Ustilago maydis,* gewonnenen Guanylat Kinase gefunden werden, können also auch mit Guanylat Kinase aus anderen zahlreichen Pilzspezies, gerade auch mit pflanzenpathogenen Pilzen interagieren, wobei die Interaktion mit den unterschiedlichen in diesen Pilzen vorkommenden Guanylat Kinase nicht immer gleich stark sein muss. Dies erklärt unter anderem die beobachtete Selektivität der an diesem Enzym wirksamen Substanzen.

Der Ausdruck "homologer Promotor", wie er hierin verwendet wird, bezieht sich auf einen Promotor, der im Ursprungsorganismus die Expression des betreffenden Gens kontrolliert. Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Der Ausdruck "Kompetitor" wie er hierin verwendet wird, bezieht sich auf die Eigenschaft der Verbindungen, mit anderen, gegebenenfalls zu identifizierenden Verbindungen um die Bindung an der Guanylat Kinase zu kompetitieren und diese vom Enzym zu verdrängen bzw. von dieser verdrängt zu werden.

Der Ausdruck "Modulator", wie er hierin verwendet wird, stellt einen Oberbegriff für Inhibitoren und Aktivatoren dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

### Beschreibung der Erfindung

In der vorliegenden Erfindung wird zum ersten Mal die vollständige Sequenz einer Guanylat Kinase aus dem pflanzenpathogenen Pilz *Ustilago maydis* zur Verfügung gestellt, die die weitere Erforschung von Guanylat Kinasen insbesondere aus pflanzenpathogenen Pilzen und damit die Erschließung eines neuen Zielproteins für die Identifizierung neuer fungizider Wirkstoffe ermöglicht.

Trotz der umfangreichen Forschung an der Guanylat Kinase war bislang unbekannt, dass die Guanylat Kinase in Pilzen ein Zielprotein (ein so genanntes "Target") fungizid wirksamer Substanzen sein kann. Damit wird in der vorliegenden Erfindung zum ersten Mal gezeigt, dass die Guanylat Kinase ein insbesondere für Pilze wichtiges Enzym darstellt und deshalb in besonderem Maße dazu geeignet ist, als Zielprotein für die Suche nach weiteren und verbesserten fungizid. wirksamen Wirkstoffen verwendet zu werden.

Inhibitoren der Guanylat Kinase mit einer fungiziden Wirkung wurden bisher noch nicht beschrieben. Zwar verweisen verschiedene Veröffentlichung auf die besondere Rolle der Guanylat Kinase für den Stoffwechsel von Organismen wie z.B. des Hefepilzes *Saccharomyces cerevisiae* und beschreiben auch, dass die Zerstörung des Hefe-Gens, welches für die Guanylat Kinase kodiert, für *S. cerevisiae* letal ist. Allerdings nimmt keine Veröffentlichung Stellung zu der Frage, ob das Enzym Guanylat Kinase durch Wirkstoffe beeinflusst, z.B. inhibiert werden kann, und ob eine Behandlung von Pilzen *in vivo* mit einem die Guanylat Kinase modulierenden Wirkstoff zur Bekämpfung von Pilzen möglich ist. Die Guanylat Kinase wurde damit als Zielprotein für Fungizide bislang noch nicht beschrieben. Es sind keine Wirkstoffe bekannt, die eine fungizide Wirkung aufweisen und deren Wirkort die Guanylat Kinase ist.

Im Rahmen der vorliegenden Erfindung konnte mit Hilfe Knock-out Experimenten gezeigt werden (Beispiel 1), dass die Guanylat Kinase bei pflanzenpathogenen Pilzen ein Angriffspunkt oder "Target" für fungizide Wirkstoffe sein kann, die Inhibition der Guanylat Kinase also zur Schädigung oder zum Absterben des Pilzes führen könnte. In weiteren Versuchen, die auf die Zugänglichkeit der Guanylat Kinase für Wirkstoffe *in vitro* und auch *in vivo* gerichtet waren, konnte das Enzym Guanylat Kinase weiter als ein Polypeptid bestimmt werden, das zum Identifizieren von Modulatoren bzw. Inhibitoren seiner enzymatischen Aktivität in geeigneten Testverfahren verwendet werden kann, was bei verschiedenen theoretisch interessanten Targets nicht selbstverständlich gegeben ist.

Im Rahmen der vorliegenden Erfindung wurde deshalb ein Verfahren entwickelt, das geeignet ist, die Aktivität der Guanylat Kinase sowie die Hemmung dieser Aktivität in einem so genannten Hemmtest zu bestimmen, auf diese Weise Inhibitoren des Enzyms z.B. in HTS- und UHTS-Verfahren zu identifizieren und deren fungizide Eigenschaften zu prüfen. Im Rahmen der vorliegenden Erfindung wurde auch gezeigt, dass die Inhibitoren der Guanylat Kinase aus Pilzen als Fungizide verwendet werden können.

Im Rahmen der vorliegenden Erfindung wurde weiter gefunden, dass die Guanylat Kinase auch *in vivo* durch Wirkstoffe inhibiert werden kann und ein mit diesen Wirkstoffen behandelter pilzlicher Organismus durch die Behandlung mit diesen Wirkstoffen geschädigt und abgetötet werden kann. Die Inhibitoren einer pilzlichen Guanylat Kinase können also als Fungizide im Pflanzenschutz oder als Antimykotika in Pharmaindikationen verwendet werden. In der vorliegenden Erfindung wird beispielsweise gezeigt, dass die Hemmung der Guanylat Kinase mit einer in einem erfindungsgemäßen Verfahren identifizierten Substanzen zum Absterben der behandelten Pilze in synthetischen Medien bzw. auf der Pflanze führt.

Die Inhibitoren einer pilzlichen Guanylat Kinase können als Fungizide insbesondere im Pflanzenschutz oder auch als Antimykotika in Pharmaindikationen verwendet werden. In der vorliegenden Erfindung wird beispielsweise gezeigt, dass die Hemmung der Guanylat Kinase mit einer in einem erfindungsgemäßen Verfahren identifizierten Substanz zum Absterben der behandelten Pilze in synthetischen Medien bzw. auf der Pflanze führt.

Guanylat Kinase kann aus verschiedenen pflanzen- oder auch human- oder tierpathogenen Pilzen gewonnen werden, z.B. aus Pilzen wie dem pflanzenpathogenen Pilz *U. maydis.* Zur Herstellung der Guanylat Kinase aus Pilzen kann das Gen z.B. rekombinant in *Escherichia coli* exprimiert und aus *E. coli* Zellen eine Enzympräparation hergestellt werden (Beispiel 2). Bevorzugt werden Guanylat Kinasen aus pflanzenpathogenen Pilzen verwendet, um im Pflanzenschutz einsetzbare Fungizide zu identifizieren. Ist das Ziel die Identifizierung von Fungiziden bzw. Antimycotika, die in Pharmaindikationen verwendet werden sollen, empfiehlt sich der Einsatz von Guanylat Kinasen aus human- bzw. tierpathogenen Pilzen.

So wurde für die Expression des von *guk1* kodierten Polypeptids GUK1 der zugehörige ORF mittels RT-PCR aus Gesamt-RNA nach dem Fachmann bekannten Methoden über ausgewählte Primer amplifiziert. Die entsprechende DNA wurde in den Vektor pDESTMC2 (Gateway Vektor von Invitrogen versehen mit einem N- terminalen MBP-Tag (= Maltose Bindeprotein Tag)) kloniert. Das resultierende Plasmid pGuk1 enthält die vollständige kodierende Sequenz von *guk1* in einer N-terminalen Fusion mit dem MBP-Tag aus dem Vektor. Das GUK1 Fusionsprotein besitzt eine berechnete Masse von ca. 64 kDa (Beispiel 2 und Abbildung 2).

Das Plasmid pGuk1 wurde dann zur rekombinanten Expression von GUK1 in *E. coli* BL21 (DE3) pLysS verwendet (Beispiel 2).

In der vorliegenden Erfindung wird damit auch eine weitere vollständige genomische Sequenz eines pflanzenpathogenen Pilzes kodierend für eine Guanylat Kinase zur Verfügung gestellt, und deren Verwendung bzw. die Verwendung des davon kodierten Polypeptids zur Identifizierung von Inhibitoren des Enzyms beschrieben.

Gegenstand der vorliegenden Erfindung ist deshalb auch die für ein Polypeptid mit der enzymatischen Funktion einer Guanylat Kinase kodierende Nukleinsäure aus dem Pilz *Ustilago maydis*.

Guanylat Kinasen teilen sich homologe Bereiche. Typisch für Guanylat Kinasen ist eine hochkonservierte Region enthaltend zwei Arginine und ein Tyrosin, die beide bei der Bindung des GMP beteiligt sind.

Diese GMP-Bindungsstelle ist ein für Guanylat Kinasen charakteristisches Sequenzmerkmal. Durch eine geeignete Suche in der PROSITE database kann ein solches Motiv identifiziert werden (Hofmann K., Bucher P., Falquet L., Bairoch A. (1999) "The PROSITE database, its status in 1999". *Nucleic Acids Res.* 27, 215). Es kann wie folgt dargestellt werden:

T-[ST]-R-x(2)-[KR]-x(2)-[DE]-x(2)-G-x(2)-Y-x-[FY]-[LIVMK],

PROSITE ermöglicht Polypeptiden eine Funktion zuzuordnen und somit Guanylat Kinasen als solche zu erkennen.

Bei der Darstellung des Prosite Motivs wird der "Ein-Buchstaben-Code" verwendet. Das Symbol "x" steht für eine Position, an der jede Aminosäure akzeptiert wird. Eine variable Position, an der verschiedene bestimmte Aminosäuren akzeptiert werden, wird in eckigen Klammern "[...]" dargestellt, wobei die an dieser Position möglichen Aminosäuren aufgezählt werden. Aminosäuren, die an einer bestimmten Position nicht akzeptiert werden, stehen dagegen in geschweiften Klammern "{...}". Ein Gedankenstrich "-" trennt die einzelnen Elemente bzw. Positionen des Motivs. Wiederholt sich eine bestimmte Position, z.B. "x", mehrfach hintereinander, kann dies durch Angabe der Zahl der Wiederholungen in einer nachfolgenden Klammer dargestellt werden, z.B. "x (3)", was für "x-x-x" steht.

Ein Prosite Motiv stellt also letztlich die Komponenten einer Konsensussequenz dar, sowie Abstände zwischen den beteiligten Aminosäuren und ist damit typisch für eine bestimmte Enzymklasse. Anhand dieses Motivs können auf Basis der erfindungsgemäßen Nukleinsäuren weitere Polypeptide aus pflanzenpathogenen Pilzen indentifiziert bzw. zugeordnet werden, die zur selben Klasse wie das erfindungsgemäße Polypeptid gehören und deshalb auch in erfindungsgemäßer Weise verwendet werden können.

Im Falle der Guanylat Kinase aus *U. maydis* liegt dieses Motiv ebenso vor wie bei *S. cerevisiae, S. pombe* oder *N. crassa* (vgl. Abbildung 1). Die spezifische Konsensussequenz für eine erfindungsgemäße Guanylat Kinase, die zur Identifizierung bzw. Zuordnung weiterer erfindungsgemäßer Polypeptide genutzt werden kann, ist deshalb besonders bevorzugt

T-T-R-x(2)-R-x(2)-E-x(2)-G-x(2)-Y-x-Y-V.

Das oben genannte Prosite Motiv bzw. die spezifische Konsensussequenz sind typisch für die erfindungsgemäßen Polypeptide, die anhand dieser Konsensussequenzen strukturell definiert werden können und damit auch eindeutig identifizierbar sind.

Gegenstand der vorliegenden Erfindung sind deshalb auch Polypeptide aus pflanzenpathogenen Pilzen mit der biologischen Aktivität einer Guanylat Kinase, die das vorstehend genannte Prosite Motiv T-[ST]-R-x(2)-[KR]-x(2)-[DE]-x(2)-G-x(2)-Y-x-[FY]-[LIVMK] umfassen, bevorzugt solche Polypeptide, die das vorstehend genannte Motiv T-T-R-x(2)-R-x(2)-E-x(2)-G-x(2)-Y-x-Y-V umfassen.

Aufgrund der Homologien, die bei speziesspezifischen Nukleinsäuren kodierend für Guanylat Kinasen vorliegen, können auch Guanylat Kinasen aus anderen pflanzenpathogenen Pilzen identifiziert und verwendet werden, um die oben gestellte Aufgabe zu lösen, d.h. sie können ebenfalls zum Identifizieren von Inhibitoren einer Guanylat Kinase verwendet werden, welche wiederum als Fungizide im Pflanzenschutz verwendet werden können. Es ist jedoch auch denkbar einen anderen Pilz, der nicht pflanzenpathogen ist, bzw. dessen Guanylat Kinase oder die dafür kodierende Sequenz zu verwenden, um fungizid wirkende Inhibitoren der Guanylat Kinase zu identifizieren. Aufgrund der hier angegebenen Sequenz gemäß SEQ ID NO: 1 und eventuell davon abgeleiteten Primern sowie gegebenenfalls unter Zuhilfenahme des vorstehend gezeigten Prosite Motivs ist es dem Fachmann möglich, z.B. mittels PCR weitere für Guanylat Kinasen kodierende Nukleinsäuren aus anderen (pflanzenpathogenen) Pilzen zu erhalten und zu identifizieren. Solche Nukleinsäuren und deren Verwendung in Verfahren zum Identifizieren von fungiziden Wirkstoffen werden als von der vorliegenden Erfindung umfasst betrachtet.

Mit Hilfe der erfindungsgemäßen Nukleinsäuresequenz sowie gemäß den vorstehend beschriebenen Verfahren erhaltene Sequenzen aus anderen pflanzenpathogenen Pilzen können weitere für eine Guanylat Kinase kodierende Nukleinsäuresequenzen aus anderen Pilzen identifiziert werden.

Gegenstand der vorliegenden Erfindung sind daher Nukleinsäuren aus pflanzenpathogenen Pilzen, die für ein Polypeptid mit der enzymatischen Aktivität einer Guanylat Kinase kodieren, insbesondere Polypeptide die die vorstehend beschriebenen Motive umfassen.

Bevorzugt sind Gegenstand der vorliegenden Erfindung Nukleinsäuren aus den vorstehend unter Defintionen genannten pflanzenpathogenen Pilzspezies, die für ein Polypeptid mit der enzymatischen Aktivität einer Guanylat Kinase kodieren.

Gegenstand der vorliegenden Erfindung ist insbesondere bevorzugt die für die Guanylat Kinase aus *Ustilago maydis* kodierende Nukleinsäure mit der SEQ ID NO:1 sowie die für die Polypeptide gemäß SEQ ID NO:2 oder aktive Fragmente davon kodierenden Nukleinsäuren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, und cDNAs.

Besonders bevorzugt umfassen die erfindungsgemäßen Nukleinsäuren eine Sequenz aus pflanzenpathogenen Pilzen kodierend für ein Polypeptid mit der enzymatischen Aktivität einer Guanylat Kinase ausgewählt aus
a) einer Sequenz gemäß SEQ ID NO: 1,
b) Sequenzen, die für ein Polypeptid kodieren, welches die Aminosäuresequenz gemäß SEQ ID NO: 2 umfasst,
c) Sequenzen, die für ein Polypeptid kodieren, welches das Motiv T-[ST]-R-x(2)-[KR]-x(2)-[DE]-x(2)-G-x(2)-Y-x-[FY]-[LIVMK] oder das Motiv T-T-R-x(2)-R-x(2)-E-x(2)-G-x(2)-Y-x-Y-V umfasst,
d) Sequenzen, welche an die unter a) und b) definierten Sequenzen bei einer Hybridisierungstemperatur von 42-65°C hybridisieren und
e) Sequenzen, welche eine zumindest 80 %ige, bevorzugt zumindest 85 %ige und besonders bevorzugt eine zumindest 90 %ige Identität mit den unter a) und b) definierten Sequenzen aufweisen.

Wie bereits vorstehend ausgeführt, ist die vorliegende Erfindung nicht nur auf die Guanylat Kinase aus *Ustilago maydis* beschränkt. In analoger und dem Fachmann bekannter Weise können auch aus anderen Pilzen, vorzugsweise aus pflanzenpathogenen Pilzen, Polypeptide mit der Aktivität einer Guanylat Kinase gewonnen werden, die dann z.B. in einem erfindungsgemäßen Verfahren eingesetzt werden können. Bevorzugt wird allerdings die Guanylat Kinase aus *Ustilago maydis* verwendet.

Gegenstand der vorliegenden Erfindung sind weiterhin DNA-Konstrukte, die eine erfindungsgemäße Nukleinsäure und einen homologen oder heterologen Promotor umfassen.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die T3-, T7- oder SP6-Promotoren für prokaryotische Zellen oder zellfreie Systeme.

Bevorzugt sollten pilzliche Expressionssysteme wie z.B. das *Pichia pastoris*-System verwendet werden, wobei hier die Transkription durch den mit Methanol induzierbaren AOX-Promotor angetrieben wird.

Gegenstand der vorliegenden Erfindung sind ferner Vektoren, die eine erfindungsgemäße Nukleinsäure, eine erfindungsgemäße regulatorische Region oder ein erfindungsgemäßes DNA-Konstrukt enthalten. Als Vektoren können alle in molekularbiologischen Laboratorien verwendeten Phagen, Plasmide, Phagmide, Phasmide, Cosmide, YACs, BACs, künstliche Chromosomen oder Partikel, die für einen Partikelbeschuss geeignet sind, verwendet werden.

Bevorzugte Vektoren sind z.B. die p4XXprom. Vektorserie (Mumberg et al., 1995) für Hefezellen, pSPORT-Vektoren (Fa. Life Technologies) für bakterielle Zellen, oder den Gateway Vektoren (Fa. Life Technologies) für verschiedene Expressionssysteme in bakteriellen Zellen, Pflanzen, P. *pastoris*, *S. cerevisiae* oder Insektenzellen.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, vorzugsweise *E. coli*, als auch eukaryotische Zellen, wie Zellen von *Saccharomyces cerevisiae, Pichia pastoris*, Insekten, Pflanzen, Froschoozyten und Zelllinien von Säugern.

Gegenstand der vorliegenden Erfindung sind weiterhin Polypeptide mit der biologischen Aktivität einer Guanylat Kinase, die von den erfindungsgemäßen Nukleinsäuren kodiert werden.

Bevorzugt umfassen die erfindungsgemäßen Polypeptide eine Aminosäuresequenz aus pflanzenpathogenen Pilzen ausgewählt aus
(a) der Sequenz gemäß SEQ ID NO:2,
(b) Sequenzen, welche eine zumindest 80 %ige, bevorzugt eine zumindest 85 %ige, besonders bevorzugt eine 90 %ige und insbesondere bevorzugt eine 95 %ige Identität mit der unter a) definierten Sequenz haben,
(c) den unter b) angegebenen Sequenzen, welche das Motiv T-[ST]-R-x(2)-[KR]-x(2)-[DE]-x(2)-G-x(2)-Y-x-[FY]-[LIVMK] oder das Motiv T-T-R-x(2)-R-x(2)-E-x(2)-G-x(2)-Y-x-Y-V umfassen, und
(d) Fragmenten der unter a) bis c) angegebenen Sequenzen, welche die gleiche biologische Aktivität aufweisen wie die unter a) definierte Sequenz.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können. In den erfindungsgemäßen Verfahren können ebenso aktive Fragmente einer Guanylat Kinase eingesetzt werden, solange sie die Bestimmung der enzymatischen Aktivität des Polypeptids bzw. deren Inhibition durch eine Kandidatenverbindung ermöglichen.

Die in den erfindungsgemäßen Verfahren eingesetzten Polypeptide können im Vergleich zu den entsprechenden Regionen natürlich vorkommender Guanylat Kinasen Deletionen oder Aminosäuresubstitutionen aufweisen, solange sie zumindest noch die biologische Aktivität einer vollständigen Guanylat Kinase zeigen. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Ein mögliches Reinigungsverfahren der Guanylat Kinase basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie (vgl. Beispiel 2).

Ein schnelles Verfahren zum Isolieren von Guanylat Kinasen, die von Wirtszellen synthetisiert werden, beginnt mit der Expression eines Fusionsproteins, wobei der Fusionspartner auf einfache Weise affinitätsgereinigt werden kann. Der Fusionspartner kann beispielsweise ein MBP-Tag sein (vgl. Beispiel 2). Das Fusionsprotein kann dann an Amylose-Resin gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Das Verfahren zum Herstellen von Polypeptiden mit der Aktivität einer Guanylat Kinase, wie z.B. des Polypeptids GUK1, ist damit gekennzeichnet durch
(a) das Kultivieren einer Wirtszelle enthaltend zumindest eine exprimierbare Nukleinsäuresequenz kodierend für ein Polypeptid aus Pilzen mit der biologischen Aktivität einer Guanylat Kinase unter Bedingungen, die die . Expression dieser Nukleinsäure gewährleisten, oder
(b) das Exprimieren einer exprimierbaren Nukleinsäuresequenz kodierend für ein Polypeptid aus Pilzen mit der biologischen Aktivität einer Guanylat Kinase in einem *in vitro*-System, und
(c) die Gewinnung des Polypeptids aus der Zelle, dem Kulturmedium oder dem *in vitro*-System.

Die so erhaltenen Zellen enthaltend das erfindungsgemäße Polypeptid oder das so erhaltene gereinigte Polypeptid sind geeignet, in Verfahren zum Identifizieren von Modulatoren bzw. Inhibitoren der Guanylat Kinase verwendet zu werden.

Gegenstand der vorliegenden Erfindung ist auch die Verwendung von Polypeptiden aus Pilzen, welche zumindest eine biologische Aktivität einer Guanylat Kinase ausüben, in Verfahren zum Identifizieren von Fungiziden, wobei die Inhibitoren der Guanylat Kinase als Fungizide verwendet werden können. Besonders bevorzugt wird die Guanylat Kinase aus *Ustilago maydis* verwendet.

Fungizide Wirkstoffe, die mit Hilfe einer Guanylat Kinase aus einer bestimmten Pilzspezies gefunden werden, können auch mit Guanylat Kinase aus anderen Pilzspezies interagieren, wobei die Interaktion mit den unterschiedlichen in diesen Pilzen vorkommenden Guanylat Kinase nicht immer gleich stark sein muss. Dies erklärt unter anderem die Selektivität wirksamer Substanzen. Die Nutzung der Wirkstoffe, die mit einer spezifischen Guanylat Kinase gefunden wurden, als Fungizid auch bei anderen Pilzen kann darauf zurückgeführt werden, dass sich Guanylat Kinase aus verschiedenen Pilzspezies sehr nahe stehen und in größeren Bereichen eine ausgeprägte Homologie zeigen. So wird an Abbildung 1 deutlich, dass eine solche Homologie über beträchtliche Sequenzabschnitte hinweg zwischen *S. cerevisiae, N. crassa, S. pombe* und *U. maydis* besteht und damit die Wirkung der z.B. mit Hilfe der Guanylat Kinase aus *U. maydis* gefundenen Substanzen nicht auf *U. maydis* beschränkt bleibt.

Gegenstand der vorliegenden Erfindung ist deshalb auch ein Verfahren zum Identifizieren von Fungiziden durch Testen von potentiellen Inhibitoren bzw. Modulatoren der enzymatischen Aktivität der Guanylat Kinase (Kandidatenverbindung) in einem Guanylat Kinase-Hemmtest.

Verfahren, die geeignet sind, Modulatoren, insbesondere Inhibitoren bzw. Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren (*in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden. Im Anschluss an die *in vivo* oder *in vitro* Identifizierung von Modulatoren des Polypeptids können Tests an Pilzkulturen durchgeführt werden, um die fungizide Wirksamkeit der gefundenen Verbindungen zu prüfen.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semi-gereinigtes Protein. Sie sind geeignet für eine "erste" Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z.B. hinsichtlich Wirksamkeit, Toxizität oder Selektivität optimiert sind.

Um Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat oder Liganden der Polypeptide in Gegenwart und Abwesenheit eines Kandidatenmoleküls inkubiert werden. Die Fähigkeit des Kandidatenmoleküls die enzymatische Aktivität der erfindungsgemäßen Polypeptide zu hemmen, wird z.B. erkennbar an einer verringerten Bindung des gegebenenfalls markierten Liganden oder an einer verringerten Umsetzung des gegebenenfalls markierten Substrates. Moleküle, die die biologische Aktivität der erfindungsgemäßen Polypeptide hemmen, sind gute Antagonisten bzw. Inhibitoren.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorimetrische nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität des Antagonisten ermessen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden.

Durch die anhand der vorliegenden Erfindung verfügbaren, für eine erfindungsgemäße Guanylat Kinase kodierende Nukleinsäuren enthaltenden Wirtszellen, wird die Entwicklung von Testsystemen, die auf Zellen basieren, zur Identifizierung von Substanzen ermöglicht, die die Aktivität der erfindungsgemäßen Polypeptide modulieren.

Vorzugsweise handelt es sich bei den zu identifizierenden Modulatoren um kleine organisch-chemische Verbindungen.

Ein Verfahren zum Identifizieren einer Verbindung, die die Aktivität einer Guanylat Kinase aus Pilzen moduliert und die als Fungizid im Pflanzenschutz verwendet werden kann, besteht bevorzugt darin, dass man
a) ein erfindungsgemäßes Polypeptid oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,
b) die Aktivität des erfindungsgemäßen Polypeptids bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und
c) die chemische Verbindung auswählt, die die Aktivität des erfindungsgemäßen Polypeptids spezifisch moduliert, und gegebenenfalls
d) die fungizide Wirkung der ausgewählten Verbindung *in vivo* prüft.

Besonders bevorzugt wird dabei diejenige Verbindung bestimmt, die die Aktivität des erfindungsgemäßen Polypeptids spezifisch inhibiert. Der Begriff "Aktivität", wie er hier verwendet wird, bezieht sich auf die biologische Aktivität des erfindungsgemäßen Polypeptids.

In einer bevorzugten Ausführungsform wird die Tatsache genutzt, dass bei der Reaktion der Guanylat Kinase ein Molekül Adenosindiphosphat (ADP) freigesetzt wird. Die Aktivität bzw. die Ab- oder Zunahme der Aktivität des erfindungsgemäßen Polypeptids kann deswegen durch den Nachweis des entstehenden ADP bestimmt werden. Dabei wird die geringere bzw. inhibierte Aktivität des erfindungsgemäßen Polypeptids anhand des Nachweises des entstehenden ADP durch Kopplung an die nachgeschaltete Reaktion der Pyruvat Kinase und Lactat Dehydrogenase verfolgt. Die Pyruvatkinase setzt dabei Phosphoenolpyruvat zu Pyruvat um, welches dann von der Lactat Dehydrogenase zur Oxidation von NADH zu NAD verwendet wird. Die durch die gekoppelte Reaktion steigende NAD-Konzentration bzw. abnehmende NADH-Konzentration kann photospektrometrisch durch Absorptions- oder Fluoreszenzmessung (Absorptionsabnahme bei 340 nm bzw. Fluoreszenzabnahme bei 340 nm (Emmision bei 465 nm)) bestimmt werden. Dabei wird die geringere bzw. inhibierte Aktivität des erfindungsgemäßen Polypeptids anhand der photospektrometrischen Bestimmung der Abnahme bzw. der Zunahme des umgesetzten NADH verfolgt.

Die Messung kann auch in für HTS- oder UHTS-Assays gängigeren Formaten erfolgen, z.B. in Mikrotiterplatten, in denen z.B. ein Gesamtvolumen von 5 bis 50 µl pro Ansatz bzw. pro Well vorgelegt wird und die einzelnen Komponenten in einer der vorstehend angegebenen Endkonzentrationen vorliegen (vgl. Beispiel 3). Dabei wird die zu testende, potentiell die Aktivität des Enzyms inhibierende oder aktivierende Verbindung (Kandidatenmolekül) z.B. in einer geeigneten Konzentration in Testpuffer enthaltend ATP, GMP, PEP, NADH sowie die Hilfsenzyme Pyruvat Kinase und Lactat Dehydrogenase vorgelegt. Dann wird das erfindungsgemäße Polypeptid im oben genannten Testpuffer zugegeben und die Reaktion damit gestartet. Der Ansatz wird dann z.B. bis zu 30 Minuten bei einer geeigneten Temperatur inkubiert und z.B. die Fluoreszenzabnahme (Extinktion 340nm; Emmision 465 nm) gemessen.

Eine weitere Messung erfolgt in einem entsprechenden Ansatz, jedoch ohne Zugabe eines Kandidatenmoleküls und ohne Zugabe eines erfindungsgemäßen Polypeptids (Negativkontrolle). Eine weitere Messung erfolgt wiederum bei Abwesenheit eines Kandidatenmoleküls, jedoch bei Anwesenheit des erfindungsgemäßen Polypeptids (Positivkontrolle). Negativ- und Positivkontrolle ergeben damit die Vergleichswerte zu den Ansätzen bei Anwesenheit eines Kandidatenmoleküls.

Um optimale Bedingungen für ein Verfahren zum Identifizieren von Inhibitoren der Guanylat Kinase bzw. zur Bestimmung der Aktivität der erfindungsgemäßen Polypeptide zu ermitteln, kann es vorteilhaft sein, den jeweiligen K_{M}-Wert des verwendeten erfindungsgemäßen Polypeptids zu bestimmen. Dieser gibt Aufschluss über die bevorzugt zu verwendende Konzentration des bzw. der Substrate. Im Fall der Guanylat Kinase aus *U. maydis* konnte ein K_{M} von 40 µM für GMP und ein K_{M} von 100 µM für ATP bestimmt werden (Abbildung 3).

Mit Hilfe der vorstehend beispielhaft beschriebenen Verfahren konnten Verbindungen identifiziert werden, die die pilzliche Guanylat Kinase inhibieren.

Es versteht sich von selbst, dass neben den genannten Verfahren zur Bestimmung der enzymatischen Aktivität einer Guanylat Kinase bzw. der Inhibition dieser Aktivität und zum Identifizieren von Fungiziden auch andere, z.B. bereits bekannte, Verfahren bzw. Hemmtests verwendet werden können, solange diese Verfahren es gestatten, die Aktivität einer Guanylat Kinase zu bestimmen und eine Inhibition dieser Aktivität durch eine Kandidatenverbindung zu erkennen.

Im Rahmen der vorliegenden Erfindung wurde auch gefunden, dass die mit Hilfe eines erfindungsgemäßen Verfahrens identifizierten Inhibitoren einer erfindungsgemäßen Guanylat Kinase geeignet sind, in einer geeigneten Formulierung Pilze zu schädigen oder zu töten.

Dazu kann z.B. in die Kavitäten von Mikrotiterplatten eine Lösung des zu prüfenden Wirkstoffs pipettiert werden. Nachdem das Lösungsmittel abgedampft war, wird zu jeder Kavität Medium hinzugefügt. Das Medium wird vorher mit einer geeigneten Konzentration von Sporen bzw. Mycel des zu prüfenden Pilzes versetzt. Die resultierenden Konzentrationen des Wirkstoffes betragen z.B. 0,1, 1, 10 und 100 ppm.

Die Platten werden anschließend auf einem Schüttler bei einer Temperatur von 22°C inkubiert, bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen kann die Wirkstoffdosis bestimmt werden, die zu einer 50 %igen Hemmung des Pilzwachstums gegenüber der unbehandelten Kontrolle führt (ED₅₀). Die vorliegende Erfindung bezieht sich daher ebenfalls auf die Verwendung von Modulatoren der Guanylat Kinase aus Pilzen, bevorzugt aus pflanzenpathogenen Pilzen als Fungizide. Die vorliegende Erfindung bezieht sich auch auf Fungizide, die mit Hilfe eines erfindungsgemäßen Verfahrens identifiziert wurden.

Verbindungen, die mit Hilfe eines erfindungsgemäßen Verfahrens identifiziert werden, und die aufgrund der Inhibition der pilzlichen Guanylat Kinase eine fungizide Wirkung aufweisen, können somit zur Herstellung von fungiziden Mitteln verwendet werden.

Die identifzierten Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Beim Einsatz der erfindungsgemäßen Verbindungen als Fungizide können die Aufwandmengen je nach Applikation innerhalb größerer Bereiche variiert werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die nachfolgenden Beispiele illustrieren verschiedene Aspekte der vorliegenden Erfindung und sind nicht limitieren auszulegen.

### Beispiele

### Beispiel 1

### Herstellung von guk1 Knock-out Mutanten in U. maydis

### Kultivierung von U. maydis

Die Stämme wurden bei 28°C auf PD-, YEPS- oder geeigneten Minimalmedien (Holliday, 1974; Tsukada et al., 1988) angezogen. Die Bildung dikaryotischer Filamente wurde nach dem Auftropfen von Stämmen auf PD-Plattenmedien, die 1 % Charcoal enthielten, beobachtet (Holliday, 1974). Pathogenitätstests wurden wie beschrieben durchgeführt (Gillessen et al., 1992). Übernachtkulturen der Stämme wurden in einer Konzentration von 4x10⁷ Zellen resuspendiert und jungen Maispflanzen (Gaspe Flint) injiziert bzw. aufgetropft. Für jeden Stamm wurden mindestens 25 Pflanzen infiziert und entstehende Tumore nach 14-21 Tagen untersucht.

### Herstellung der Knock-out Kassette

Molekularbiologische Standardmethoden wurden nach Sambrook *et al*., 1989 durchgeführt. Um *guk1*-Nullmutanten herzustellen, wurde die 5' und die 3' Flanke des *guk1*-Gens mittels PCR amplifiziert. Als Template diente geonomische DNA des Stammes UM518. Für die 5' Flanke (1347bp) wurden die Primer LB2 mit der Sequenz (5'-cacggcctgagtggcccgtttgtcgcttggaatcggaggc-3') und p15 mit der Sequenz (5'-gcgcttctgcctcacctgtgccc-3') eingesetzt. Für die 3'-Flanke (2723bp) wurden die Primer RB1 (5'-gtgggccatctaggcccctcgttcactagccgacaacgcc-3') und p13 (5'-ccaacgtagaggccgcagaagacc-3') verwendet. Mit den Primem LB2 und RB1 wurden die Schnittstellen *Sfi* I (a) und *Sfi* I (b) eingeführt. Die Amplikons wurden mit *Sfi* I restringiert und mit dem 1884 bp großen *Sfi* I-Fragment aus dem Vektor pBS-isoliert (HygromycinB-Kassette) ligiert. Durch eine PCR mit den Primem LB1 (5'-cggacttgaggagaccatcatagcc-3') und RB2 (5'-ccctgcatgctgagcagacatgcc-3') wurde die 4322 bp große *guk1*-Knock-out Kassette amplifiziert, die in die nachfolgende Transformation eingesetzt wurde (Kämper und Schreier, 2001).

### Herstellung von Protoplasten von U. maydis

50 ml einer Kultur in YEPS Medium wurden bei 28°C bis zu einer Zelldichte von ca. 5x10⁷/ml (OD 0.6 bis 1.0) angezogen und dann für 7 min. bei 2500 g (Hereaus, 3500 rpm) in 50 ml Falkonröhrchen abzentrifugiert. Das Zellpellet wurde in 25 ml SCS-Puffer (20 mM NaCitrat pH 5.8, 1.0 M Sorbit, (20 mM NaCitrat/1.0 M Sorbit und 20 mM Zitronensäure/1.0 M Sorbit mischen und mit pH-Meter auf pH 5.8 einstellen)) resuspendiert, erneut 7 min. bei 2500 g (3500 rpm) zentrifugieren und das Pellet in 2 ml SCS-Puffer, pH 5,8, mit 2,5 mg/ml Novozym 234 resuspendiert. Die Protoplastierung erfolgte bei Raumtemperatur und wurde mikroskopisch alle 5 min. verfolgt. Die Protoplasten wurden dann mit 10 ml SCS-Puffer gemischt und bei 1100 g (2300 rpm) 10 min. zentrifugiert, der Überstand wurde verworfen. Das Pellet wurde vorsichtig in 10 ml SCS Puffer resuspendiert und erneut zentrifugiert. Der Waschvorgang mit SCS-Puffer wurde zweimal wiederholt, das Pellet wurde in 10 ml STC-Puffer gewaschen. Schließlich wurde das Pellet in 500 µl kaltem STC-Puffer (10 mM Tris/HCL pH 7.5, 1.0 M Sorbit, 100 mM CaCl₂) resuspendiert und auf Eis gehalten. Aliquots können mehrere Monate bei -80°C gelagert werden.

### Transformation von U. maydis

Die Transformation des diploiden *U. maydis* Stammes erfolgte nach Schulz *et al*., 1990. Die Isolierung genomischer *U. maydis* DNA erfolgte wie bei Hoffmann und Winston 1987 beschrieben bzw. nach dem Protokoll des Firma Qiagen (DNeasy-Kit).

Zur Transformation wurden max. 10 µl DNA (optimal 3-5 µg) in ein 2 ml Eppendorfröhrchen übertragen, 1 µl Heparin (15 µg/µl) (SIGMA H3125) zugegeben und dann 50 µl Protoplasten zugesetzt und 10 min. auf Eis inkubiert. 500 µl 40 % (w/w) PEG3350 (SIGMA P3640) in STC (steril filtriert) wurden zugesetzt, vorsichtig mit der Protoplastensuspension gemischt und 15 min. auf Eis inkubiert. Das Ausplattieren erfolgte auf Gradienten-Platten (unterer Agar: 10 ml YEPS-1,5% Agar-1M Sorbitol mit Antibiotikum; kurz vor dem Ausplattieren wurde die untere Agarschicht mit 10 ml YEPS- 1,5 % Agar-1M Sorbitol überschichtet, die Protoplasten ausgestrichen und die Platten für 3-4 Tage bei 28°C inkubiert.

Der Nachweis der homologen Rekombination in einen genomischen Lokus von guk1 wurde mittels Standardmethoden (PCR oder Southernanalyse) von isolierter genomischer DNA durchgeführt. Hierbei wurde gezeigt, dass die Integration der *guk1*-Knock-out Kassette in einen genomischen *guk1*-Lokus stattgefunden hat und somit eine Wildtypkopie des *guk1*-Gens ersetzt wurde, während die zweite Kopie in diesem diploiden Stamm erhalten blieb. Die auf diese Weise entstandenen heterozygoten *guk1* Mutanten wurden nachfolgend in den Pathogenitätstest eingesetzt (Gillessen et al., 1992).

### Sporenanalyse von U. maydis

Aus den im Pathogenitätstest erzeugten Tumoren wurden Sporen isoliert. Im Anschluss wurden die entstehenden Sporidien vereinzelt und phäno- bzw. genotypisch untersucht. Die phänotypische Analyse erfolgte mittels Wachstumsversuchen auf geeigneten Voll- bzw. Minimalmedien (Holliday, 1974; Tsukada et al., 1988). Dabei zeigte sich, das nur 9 von 75 analysierten Sporidien unter selektiven Bedingungen wuchsen. Von diesen waren 7 Stämme diploid. Dies war ein erster Hinweis darauf, dass der Phänotyp der gukl-Null-Mutante lethal war. Die genotypischen Untersuchungen erfolgten durch Southern- bzw. PCR basierter Analyse der Sporidien. Hierbei wurde gefunden, das unter 75 analysierten Sporidien kein lebensfähiger, haploider Stamm identifiziert werden konnte, in dem nur das *guk1*-Gen durch die *guk1*-Knock-out-Kassette ersetzt war. In den Fällen, in denen die *guk1*-Knock-out-Kassette homolog in den genomischen Lokus von *guk1* integriert war, wurde zusätzlich eine ektopische Kopie des Gens gefunden. Aus diesen Ergebnissen wurde geschlossen, dass der Knock-out des *guk1*-Gens in *Ustilago maydis* zu einem letalen Phänotyp führt.

### Beispiel 2

### Klonierung, Expression und Reinigung von guk1 bzw. GUK1 aus Ustilago maydis

Für die Klonierung von *guk1* bzw. dessen Expression wurde der ORF mittels RT-PCR aus Gesamt RNA aus *Ustilago maydis* über genspezifische Primer amplifiziert. Die entsprechende DNA, ein Amplikon von 609 bp Länge, wurde in den Vektor pDESTMC2 (Gateway Vektor von Invitrogen versehen mit einem N- terminalen MBP-Tag (= Maltose Bindeprotein Tag)) kloniert. Das resultierende Plasmid pGuk1 enthält die vollständige kodierende Sequenz von *guk1* in einer N-terminalen Fusion mit dem MBP-Tag, das Bestandteil des Vektors ist. Das GUK1 Fusionsprotein besitzt eine berechnete Masse von 64 kDa.

Für die heterologe Expression wurde das Plasmid pGuk1 so in *E. coli* BL21 (DE3) pLysS Zellen transformiert, dass der Transformationsansatz direkt als Vorkultur in 50 ml Selektionsmedium diente. Diese Zellen wurden über Nacht bei 37°C inkubiert und anschließend 1:25 in Selektionsmedium (LB-Medium mit 100 µg/ml Ampicillin) verdünnt. Die Induktion erfolgte bei einer OD₆₀₀ₙₘ von 0,8 - 1,0 mit 1 mM IPTG (Endkonzentration) bei 28°C. Nach 4 h Induktion wurden die Zellen geerntet und bei -20°C gelagert. Für den Aufschluss wurde das Pellet in 15 ml Lysepuffer (20 mM Tris / HCl pH 7.5 ; 200 mM NaCl ; 1 mM EDTA + 600 µl Lysozym (50 mg / ml) + 150 µl DNAseI + 330 µl MgCl₂ (1M) + 1mM DTT) und anschliessend mittels Sonifizieren aufgeschlossen. Die durch Zentrifugation (15 min bei 4°C, 10 000 g) erhaltene Cytoplasmafraktion wurde für die Aufreinigung des exprimierten Proteins verwendet. Die Reinigung erfolgte nach dem Standardprotokoll des Herstellers für Amylose Resin (New England Biolabs). Das gereinigte Protein wurde in Puffer mit Glycerin versetzt (Lysepuffer + 10 mM Maltose, 10 % Glycerin) und bei -80°C gelagert.

### Beispiels 3

### Identifzierung von Modulatoren der Guanylat Kinase in 384-Well-MTP in einem gekoppelten Assay

Zur Identifizierung von Modulatoren der Guanylat Kinase wurden 384-Well-Mikrotiterplatten von Greiner verwendet.

In die erste Reihe wurde die Negativ-Kontrolle pipettiert. Diese setzte sich zusammen aus 25 µl Testpuffer (100 mM Tris/HCl pH 7,7, 15 mM MgCl₂, 100 mM KCl) und 20 µl Substratlösung (250 mM Tris/HCl, pH 7,7; 250 mM KCl; 37,5 mM MgCl₂; 1,05 mM PEP; 760 µM NADH; 850 µM GMP; 2 mM ATP; 5 U/mL Pyruvat Kinase; 5 U/mL Lactat Dehydrogenase). In die zweite Reihe wurde die Positiv-Kontrolle pipettiert. Diese setzte sich zusammen aus 5µl Testpuffer mit 5 % DMSO, und 20 µl Substratlösung (250 mM Tris/HCl, pH 7,7; 250 mM KCl; 37,5 mM MgCl₂; 1,05 mM PEP; 760 µM NADH; 850 µM GMP; 2 mM ATP; 5 U/mL Pyruvat Kinase; 5 U/mL Lactat Dehydrogenase) und 25 µl Enzymlösung (0,3 µg/mL Guanylat Kinase; 20 % Glycerol, 0,5 % BSA; 100 mM Tris/HCl pH7,7; 100 mM KCl, 15 mM MgCl₂). In die verbleibenden Reihen wurde eine Prüfsubstanz in einer Konzentration von 2 µM in DMSO vorgelegt, wobei zum Verdünnen der Substanz auf ein Volumen von 5 µl des Testpuffers verwendet wurde. Nach Zugabe von 20 µl Substratlösung (250 mM Tris/HCl, pH 7,7; 250 mM KCl; 37,5 mM MgCl₂; 1,05 mM PEP; 760 µM NADH; 850 µM GMP; 2 mM ATP; 5 U/mL Pyruvat Kinase; 5 U/mL Lactat Dehydrogenase) wurden zum Start der Reaktion 25 µl Enzymlösung (0,3 µg/mL Guanylat Kinase; 10 % Glycerol, 0,5 % BSA; 100 mM Tris/HCl pH7,7; 100 mM KCl, 15 mM MgCl₂) zugegeben.

Es folgte Inkubation bei Raumtemperatur für 30 Minuten. Anschliessend wurde das während der Reaktion umgesetzte NADH durch Bestimmung der Fluoreszenzabnahme (Extinktion 340nm; Emmision 465 nm) in einem für MTP geeigneten SPECTRAFluor Plus von Tecan gemessen.

### Beispiel 4

### Nachweis der fungiziden Wirkung der identifizierten Inhibitoren der Guanylat Kinase

In die Kavitäten von Mikrotiterplatten werden eine methanolische Lösung des anhand eines erfindungsgemäßen Verfahrens identifizierten Wirkstoffs (Bsp. 3), versetzt mit einem Emulgator, pipettiert. Nachdem das Lösungsmittel abgedampft ist, werden je Kavität 200 µl Potatoe-Dextrose-Medium hinzugefügt. Das Medium wird vorher mit geeigneten Konzentrationen von Sporen bzw. Mycelen des zu prüfenden Pilzes versetzt.

Die resultierenden Konzentrationen des Wirkstoffs beträgt 0,1, 1, 10 und 100 ppm. Die resultierende Konzentration des Emulgators beträgt 300 ppm.

Die Platten werden anschließend auf einem Schüttler bei einer Temperatur von 22°C inkubiert, bis in der unbehandelten Kontrolle ein ausreichendes Wachstum feststellbar ist. Die Auswertung erfolgt photometrisch bei einer Wellenlänge von 620 nm. Aus den Messdaten der verschiedenen Konzentrationen wird die Wirkstoffdosis, die zu einer 50 %igen Hemmung des Pilzwachstums gegenüber der unbehandelten Kontrolle führt (ED₅₀), berechnet.

### Literatur

Berger, Albin; Schiltz, Emile; Schulz, Georg E. Guanylate kinase from Saccharomyces cerevisiae. Isolation and characterization, crystallization and preliminary x-ray analysis, amino acid sequence and comparison with adenylate kinases. European Journal of Biochemistry (1989), 184(2), 433-43.

Blaszczyk, Jaroslaw; Li, Yue; Yan, Honggao; Ji, Xinhua. Crystal Structure of Unligated Guanylate Kinase from Yeast Reveals GMP-induced Conformational Changes. Journal of Molecular Biology (2001), 307(1), 247-257.

Gillissen, B., Bergemann, J., Sandmann, C. Schroeer, M., Bölker, M., und Kahmann, R. (1992), A two-component regulatory system for self/nonself recognition in *Ustilago maydis.* Cell, 68: 647-657.

Hoffman, Charles S.; Winston, Fred. A ten-minute DNA preparation from yeast efficiently releases autonomous plasmids for transformation of Escherichia coli. Gene (1987), 57(2-3), 267-72.

Hofmann K., Bucher P., Falquet L., Bairoch A. (1999) The PROSITE database, its status in 1999. *Nucleic Acids Res.* 27, 215.

Holliday, R. (1974), *Ustilago maydis.* In King, R. C. (ed), Handbook of Genetics. Plenum, New York, pp. 575-595.

Kämper und Schreier (2001), Verfahren zur Herstellung von Deletionsmutanten; Deutsche Patentanmeldung Nr. 10 133 926.3.

Konrad, Manfred. Cloning and expression of the essential gene for guanylate kinase from yeast. Journal of Biological Chemistry (1992), 267(36), 25652-5.

Li, Yue; Yanling, Zhang; Yan, Honggao. Kinetic and thermodynamic characterizations of yeast guanylate kinase. Journal of Biological Chemistry (1996), 271(45), 28038-28044.

Moriguchi, Mitsuaki; Kohno, Hirao; Kamei, Masaharu; Tochikura, Tatsurokuro. Purification and properties öf guanylate kinase from bakers' yeast. Biochimica et Biophysica Acta (1981), 662(1), 165-7.

Mumberg, D., Müller, R., Funk, M.,.1995. Yeast vectors for the controlled expression of heterologous proteins in different genetic backgrounds. Gene 156, 119-122.

Sambrook, J., Fritsch, E., F. und Maniatis, T. (1989), Molecular cloning: A laboratory manual. Cold spring harbor laboratory press, Cold spring harbor, New York.

Schulz, B., Banuett, F., Dahl, M., Schlesinger, R., Schäfer, W., Martin, T., Herskowitz, I. und Kahmann, R. (1990), The *b* alleles of *Ustilago maydis*, whose combinations program pathogenic development, code for polypeptides containing a homeodomain-related motif. Cell 60, 295-306.

Stehle, Thilo; Schulz, Georg E. Three-dimensional structure of the complex of guanylate kinase from yeast with its substrate GMP. Journal of Molecular Biology (1990), 211(1), 249-54.

Tsukuda, T. et al. (1988) Isolation and characterization of an autonomously replicating sequence from *Ustilago maydis*. Mol. Cell. Biol. 8: 3703-3709.

## Patentansprüche

1. Verfahren zum Identifizieren von Fungiziden, **dadurch gekennzeichnet, dass** man
(a) ein pilzliches Polypeptid mit der enzymatischen Aktivität einer Guanylat Kinase mit einer chemischen Verbindung oder einem Gemisch von chemischen Verbindungen unter Bedingungen, die die Interaktion der chemischen Verbindung mit dem Polypeptid erlauben, in Kontakt bringt,
(b) die Aktivität der Guanylat Kinase bei Abwesenheit einer chemischen Verbindung mit der Aktivität der Guanylat Kinase bei Anwesenheit einer chemischen Verbindung oder eines Gemisches von chemischen Verbindungen vergleicht, und
(c) die chemische Verbindung, die die Guanylat Kinase spezifisch inhibiert, auswählt.

2. Verfahren gemäß Anspruch 1, **dadurch gekennzeichnet, dass** man
(a) das bei der Reaktion der Guanylat Kinase entstehende ADP mit Hilfe einer Pyruvatkinase zu ATP umsetzt,
(b) das dabei entstehende Pyruvat mit Hilfe einer Lactatdehydrogenase unter NADH-Verbrauch zu Lactat umsetzt, und
(c) den Verbrauch des NADHs verfolgt.

3. Verfahren gemäß Anspruch 2, **dadurch gekennzeichnet, dass** man eine Hemmung der enzymatischen Aktivität anhand einer geringeren Zunahme der ADP-Konzentration bestimmt.

4. Verfahren gemäß einem der Ansprüche 1 bis 3, **dadurch gekennzeichnet, dass** man in einem weiteren Schritt die fungizide Wirkung der identifizierten Verbindung testet, indem man sie mit einem Pilz in Kontakt bringt.

5. Verfahren gemäß einem der Ansprüche 1 bis 5, **dadurch gekennzeichnet, dass** man eine Guanylat Kinase aus einem pflanzenpathogenen Pilz verwendet.

6. Verwendung von Polypeptiden mit der Aktivität einer Guanylat Kinase zum Identifizieren von Fungiziden.

7. Verwendung von Inhibitoren von Polypeptiden mit der Aktivität einer Guanylat Kinase als Fungizide.

8. Verwendung von Inhibitoren eines Polypeptids mit der Aktivität einer Guanylat Kinase, welche durch ein Verfahren gemäß einem der Ansprüche 1 bis 5 identifiziert werden, als Fungizide.

9. Verwendung von fungiziden Verbindungen, die in einem Verfahren gemäß einem der Ansprüche 1 bis 5 gefunden werden, zum Herstellen von fungiziden Mitteln.

10. Nukleinsäure, kodierend für ein Polypeptid mit der biologischen Aktivität einer Guanylat Kinase, **dadurch gekennzeichnet, dass** sie aus einem pflanzenpathogenen Pilz stammt.

11. Nukleinsäure gemäß Anspruch 10, **dadurch gekennzeichnet, dass** sie eine Sequenz umfasst, die ausgewählt ist aus:
a) einer Sequenz gemäß SEQ ID NO: 1,
b) Sequenzen, die für ein Polypeptid kodieren, welches die Aminosäuresequenz gemäß SEQ ID NO: 2 umfasst,
c) Sequenzen, die für ein Polypeptid kodieren, welches das Motiv T-[ST]-R-x(2)-[KR]-x(2)-[DE]-x(2)-G-x(2)-Y-x-[FY]-[LIVMK] oder das Motiv T-T-R-x(2)-Rx(2)-E-x(2)-G-x(2)-Y-x-Y-V umfasst,
d) Sequenzen, welche an die unter a) und b) definierten Sequenzen bei einer Hybridisierungstemperatur von 42-65°C hybridisieren und
e) Sequenzen, welche eine zumindest 80 %ige, bevorzugt eine zumindest 85 %ige und besonders bevorzugt eine zumindest 90 %ige Identität mit den unter a) und b) definierten Sequenzen aufweisen.

12. DNA-Konstrukt umfassend eine Nukleinsäure gemäß Anspruch 10 oder 11 und einen heterologen Promotor.

13. Vektor umfassend eine Nukleinsäure gemäß Anspruch 10 oder 11, oder ein DNA-Konstrukt gemäß Anspruch 12.

14. Vektor gemäß Anspruch 13, **dadurch gekennzeichnet, dass** die Nukleinsäure funktionell mit regulatorischen Sequenzen verknüpft ist, die die Expression der Nukleinsäure in prooder eukaryotischen Zellen gewährleisten.

15. Wirtszelle enthaltend eine Nukleinsäure gemäß Anspruch 10 oder 11, ein DNA-Konstrukt gemäß Anspruch 12 oder einen Vektor gemäß Anspruch 13 oder 14.

16. Polypeptid mit der biologischen Aktivität einer Guanylat Kinase, welches von einer Nukleinsäure gemäß Anspruch 10 oder 11 kodiert wird.

17. Polypeptid mit der biologischen Aktivität einer Guanylat Kinase, welches eine Aminosäuresequenz gemäß SEQ ID NO: 2 umfasst.
